(19) Europäisches Patentamt
European Patent Office
Office européen des brevets

(11) EP 0 608 393 B1

(12) **EUROPÄISCHE PATENTSCHRIFT**

(45) Veröffentlichungstag und Bekanntmachung des Hinweises auf die Patenterteilung:
**20.03.1996 Patentblatt 1996/12**

(21) Anmeldenummer: **93915799.6**

(22) Anmeldetag: **06.07.1993**

(51) Int. Cl.[6]: **A61K 7/13**, C09B 29/08, C07C 311/38

(86) Internationale Anmeldenummer: **PCT/EP93/01734**

(87) Internationale Veröffentlichungsnummer:
**WO 94/04123 (03.03.1994 Gazette 1994/06)**

(54) **HAARFÄRBEMITTEL**
HAIR DYES
AGENTS POUR LA TEINTURE DES CHEVEUX

(84) Benannte Vertragsstaaten:
**DE ES FR GB IT**

(30) Priorität: **19.08.1992 DE 4227403**

(43) Veröffentlichungstag der Anmeldung:
**03.08.1994 Patentblatt 1994/31**

(73) Patentinhaber: **Wella Aktiengesellschaft**
**D-64274 Darmstadt (DE)**

(72) Erfinder: **BRAUN, Hans-Jürgen**
**CH-3182 Ueberstorf (CH)**

(56) Entgegenhaltungen:
**GB-A- 1 166 906** **GB-A- 2 165 257**

- **CHEMICAL ABSTRACTS, vol. 58, no. 1, 7. Januar 1963, Columbus, Ohio, US; abstract no. 1561e, SANIELEVICI ET ALL 'dyes for synthetic fibers. azoic disperse dyes with sulfonamide goups'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 608 393 B1

Printed by Rank Xerox (UK) Business Services
2.9.13/3.4

**Beschreibung**

Gegenstand der Erfindung ist ein Mittel zum Färben von Haaren mit einem Gehalt an einem 4-[4'-[Bis(β-hydroxyethyl)-amino]-phenylazo]-benzolsulfonsäureamid.

Für das Färben von Haaren gewinnen, neben den Oxidationshaarfarbstoffen, die durch oxidative Kupplung einer oder mehrerer Entwicklerkomponenten untereinander gebildet werden, in zunehmenden Maße direktziehende Haarfarbstoffe an Bedeutung. Direktziehende Farbstoffe bieten den Vorteil, daß sie ohne Zusatz von Oxidationsmitteln (beispielsweise Wasserstoffperoxid) zur Anwendung kommen und deshalb das Haar während des Färbevorganges wesentlich weniger geschädigt wird.

Gute Haarfärbemittel müssen eine Vielzahl von Anforderungen erfüllen. So müssen sie die gewünschten Farbnuancen in ausreichender Intensität ausbilden und gleichmäßig auf das Haar aufziehen, ohne die Kopfhaut zu stark anzufärben. Die erzeugten Haarfärbungen müssen weiterhin eine ausreichende Stabilität gegen Licht, Wärme, Schweiß, Haarreinigungsmittel und die bei der Dauerverformung der Haare verwendeten Chemikalien aufweisen sowie gegen die Einwirkung von sauren Mitteln und verdünnten Säuren stabil sein. Schließlich sollen diese Mittel in toxikologischer und dermatologischer Hinsicht unbedenklich sein.

Da eine gleichmäßige Haarfärbung vom Haaransatz bis in die Haarspitzen in der Regel mit Farbstoffen, die zu einer einzigen Verbindungsklasse gehören, nicht möglich ist, werden üblicherweise Kombinationen von Farbstoffen aus mehreren verschiedenen Verbindungsklassen - beispielsweise Kombinationen von Amino-, Diamino- oder Hydroxyaminonitrobenzolderivaten mit Azo- und Anthrachinonfarbstoffen - verwendet.

Der bisher am häufigsten verwendete Azofarbstoff ist das 4'-Amino-4-[bis-(β-hydroxyethyl)-amino]-azobenzol (DISPERSE BLACK 9). Ebenfalls ist es aus der DE-OS 35 34 885 bekannt, das 4'-Amino-2-methyl-4-[bis-(β-hydroxyethyl)-amino]-azobenzol als Haarfarbstoff einzusetzen.

Obwohl das 4'-Amino-4-[bis-(β-hydroxyethyl)-amino]-azobenzol häufig in Haarfärbemitteln eingesetzt wird, ist dieser Farbstoff in anwendungstechnischer Hinsicht nicht völlig zufriedenstellend. Zum einen besteht der Verdacht, daß diese Verbindung mutagen ist, und zum anderen besitzen die mit dieser Verbindung erzielten Haarfärbungen, ebenso wie die mit den aus der DE-OS 35 34 885 bekannten Haarfärbemitteln erzielten Haarfärbungen, nur eine geringe Stabilität gegenüber der Einwirkung von Säuren und sauren Zubereitungen, wie zum Beispiel sauren Haarspülungen und Shampoos, sauren Dauerwellmitteln und Fixierungen.

Es bestand daher die Aufgabe, Haarfärbemittel mit einem Gehalt an einem Azofarbstoff zur Verfügung zu stellen, die eine gute physiologische Verträglichkeit besitzen und deren Färbungen gegen Säuren und saure Zubereitungen, wie zum Beispiel saure Haarspülungen oder Haarreinigungsmittel, saure Dauerwellmittel und Fixiermittel, stabil sind und dadurch eine dauerhafte und über einen längeren Zeitraum gleichbleibende Färbung der Haare ermöglichen.

Es wurde nunmehr gefunden, daß durch ein Mittel zum Färben von Haaren mit einem Gehalt an einem 4-[4'-[Bis(β-hydroxyethyl)-amino]-phenylazo]-benzolsulfonsäureamid der Formel (I) die gestellte Aufgabe in hervorragender Weise gelöst wird.

Gegenstand der vorliegenden Erfindung ist daher ein Mittel zum Färben von Haaren mit einem Gehalt an für Haarfärbemittel üblichen Zusätzen, welches dadurch gekennzeichnet ist, daß es mindestens ein 4-[4'-[Bis-(β-hydroxyethyl)-amino]-phenylazo]-benzolsulfonsäureamid der allgemeinen Formel (I)

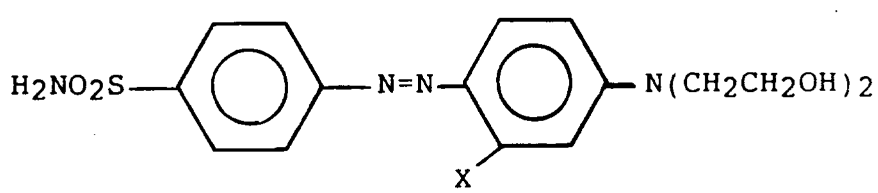

(I),

wobei X Wasserstoff, Fluor, Chlor, Brom, einen $C_1$- bis $C_3$-Alkylrest oder einen $C_1$- bis $C_3$-Alkoxyrest bedeutet, enthält.

Von den erfindungsgemäßen Mitteln ist jenes bevorzugt, welches ein 4-[4'- Bis-(β-hydroxyethyl)-amino]-phenylazo]-benzolsulfonsäureamid der Formel (I), wobei X Wasserstoff, Fluor, Chlor, Methyl oder Methoxy bedeutet, enthält. Die Farbstoffe der allgemeinen Formel (I) sollen in dem erfindungsgemäßen Mittel in einer Konzentration von 0,01 bis 5 Gewichtsprozent, vorzugsweise in einer Konzentration von 0,01 bis 2 Gewichtsprozent, enthalten sein.

Bei dem erfindungsgemäßen Haarfärbemittel handelt es sich um ein Mittel, das mindestens einen Farbstoff der allgemeinen Formel (I) enthält, oder aber um ein Mittel, das zusätzlich zu mindestens einem Farbstoff der allgemeinen Formel (I) noch einen oder mehrere weitere direkt auf das Haar aufziehende Farbstoffe enthält. Von diesen direkt auf das Haar aufziehenden Farbstoffen seien beispielsweise die folgenden erwähnt: aromatische Nitrofarbstoffe, wie zum Beispiel 2-Amino-6-chlor-4-nitrophenol, 2-Amino-4,6-dinitrophenol, 4-[(β-Hydroxyethyl)-amino]-2-nitroanilin, 2-Chlor-6-ethylamino-4-nitro-benzol, $N^1,N^4,N^4$-Tris(β-hydroxyethyl)-p-phenylendiamin (HC BLUE 2), 4-[Bis-(β-hydroxyethyl)-

amino]-1-methylamino-2-nitrobenzol, 1-[(2,3-Dihydroxypropyl)amino]-4-ethyl-[(2-hydroxyethyl)amino]-2-nitrobenzol, 1-[(2,3-Dihydroxypropyl)amino]-4-dimethylamino-2-nitrobenzol, 1-[(2,3-Dihydroxypropyl)amino]-2-nitro-4-pyrrolidinobenzol, 4-[Bis-(β-hydroxyethyl)amino]-1-[(3-hydroxypropyl)amino]-2-nitrobenzol, 2-Amino-4-nitrophenol, 2-Nitro-1,4-diaminobenzol, 2-Amino-5-nitrophenol, 4-(β-Hydroxyethyl)amino-3-nitrophenol, 1-(β-Hydroxyethyl)amino-2-amino-4-nitrobenzol, 4-(β-Ureidoethyl)-amino-nitrobenzol, 4-(2',3'-Dihydroxypropyl)-amino-3-nitrotrifluormethylbenzol, 1,4-Bis-[(β-hydroxyethyl)-amino]-4-N-ethyl-2-nitrobenzol, 4-(β-Hydroxyethyl)-amino-3-nitrotoluol, 2,5-Bis-[(β-hydroxyethyl)amino]-nitrobenzol, 2-(β-Hydroxyethyl)amino-4,6-dinitrophenol,1-Amino-4-(2',3'-dihydroxypropyl)amino-2-nitro-5-chlorbenzol, 1-Amino-2-nitro-4-[bis-(β-hydroxyethyl)amino]-benzol; Triphenylmethanfarbstoffe, wie zum Beispiel Basic Violet 1 (C.I. 42 535), Basic Violet 14 (C.I. 42 510), Basic Violet 2 (C.I. 42 520); Azofarbstoffe, wie beispielsweise Acid Brown 4 (C.I. 14 805); Anthrachinonfarbstoffe, wie zum Beispiel 1-[(β-Hydroxyethyl)-amino]-4-methylamino-anthrachinon (DISPERSE BLUE 3; C.I. 61 505), DISPERSE BLUE 23 (C.I. 61 545), 1,4-Diamino-anthrachinon (DISPERSE VIOLET 1; C.I. 61 100), 1,4,5,8-Tetraaminoanthrachinon (C.I. 64 500), wobei die Farbstoffe dieser Klassen je nach Art ihrer Substituenten sauren, nichtionogenen oder basischen Charakter haben können. Weitere geeignete, direkt auf das Haar aufziehende Farbstoffe sind beispielsweise in dem Buch von J. C. Johnson, "Hair Dyes", Noyes Data Corp., Park-Ridge (USA) (1973), Seiten 3-91 und 113-139 (ISBN: 0-8155-0477-2) beschrieben.

Der Gesamtgehalt an Haarfarbstoffen soll vorzugsweise 0,01 bis 8,0 Gewichtsprozent betragen.

Die Zubereitungsform des hier beschriebenen Haarfärbemittels, welches häufig auch als Tönungsmittel bezeichnet wird, kann beispielsweise die einer Lösung, insbesondere einer wäßrigen oder wäßrig-alkoholischen Lösung, sein. Bevorzugte Zubereitungsformen sind weiterhin die einer Creme, eines Geles, einer Emulsion oder die eines Schaumes, wobei sie auch im Gemisch mit einem Treibgas oder mittels einer Pumpe versprüht werden können.

Der pH-Wert dieses Färbemittels liegt im Bereich von 3 bis 12, insbesondere bei pH 8 bis 11,5, wobei die Einstellung des gewünschten alkalischen pH-Wertes hauptsächlich mit Ammoniak erfolgt, jedoch auch mit organischen Aminen wie beispielsweise Monoethanolamin oder Triethanolamin vorgenommen werden kann.

Die Verwendung des hier beschriebenen Haarfärbemittels erfolgt überlicherweise durch Aufbringen einer für die Haarfärbung ausreichenden Menge des Mittels auf die Haare, mit denen es 5 bis 30 Minuten lang in Berührung bleibt.

Anschließend wird mit Wasser, gegebenenfalls noch mit einer wäßrigen Lösung einer schwachen organischen Säure, gespült und sodann getrocknet.

Als schwache organische Säure können beispielsweise Essigsäure, Zitronensäure, Weinsäure und ähnliche Verwendung finden.

Das erfindungsgemäße Haarfärbemittel kann in Form eines Haarfärbemittels mit zusätzlicher Haarfestigung vorliegen, das mindestens ein in der Kosmetik üblicherweise verwendetes Polymerisat oder natürliches Polymer enthält. Solche Mittel werden im allgemeinen als Tönungsfestiger oder Farbfestiger bezeichnet.

Von den für diesen Zweck in der Kosmetik bekannten Polymerisaten seien beispielsweise Polyvinylpyrrolidon, Polyvinylacetat, Polyvinylalkohol oder Polyacrylverbingungen wie Polyacrylsäure beziehungsweise Polymethacrylsäure, basische Polymerisate der Ester der Polyacrylsäure oder Polymethracylsäure mit Aminoalkoholen beziehungsweise deren Salze oder Quaternisierungsprodukte, Polyacrylnitril, Polyvinyllactame sowie Copolymerisate aus diesen Verbindungen wie zum Beispiel Polyvinylpyrrolidon-Vinylacetat und dergleichen erwähnt.

Auch natürliche Polymere, wie zum Beispiel Chitosan (entacetyliertes Chitin) oder Chitosanderivate, können für den genannten Zweck eingesetzt werden.

Die Polymerisate und natürlichen Polymere sind in dem zuvor beschriebenen Haarfärbemittel in der für solche Mittel üblichen Menge von 1 bis 5 Gewichtsprozent enthalten. Der pH-Wert des Mittels liegt im Bereich von etwa 6,0 bis 9,0. Die Anwendung dieses Haarfärbemittels mit zusätzlicher Festigung erfolgt in bekannter und üblicher Weise durch Befeuchten des Haares mit dem Festiger, Festlegen (Einlegen) des Haares zur Frisur und anschließende Trocknung.

Selbstverständlich kann das vorstehend beschriebene Haarfärbemittel gegebenenfalls weitere, für Haarfärbemittel übliche Zusätze, wie zum Beispiel Konservierungsstoffe und Parfümöle, Lösungsmittel wie Wasser, niedere aliphatische Alkohole, beispielsweise Ethanol, Propanol und Isopropanol oder Glykole wie Glycerin und 1,2-Propylenglykol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, weiterhin Vaseline, Paraffinöl und Fettsäuren sowie außerdem Pflegestoffe wie kationische Harze, Lanolinderivate, Cholesterin, Pantothensäure und Betain, enthalten. Die erwähnten Bestandteile werden in den für solche Zwecke üblichen Mengen verwendet, zum Beispiel die Netzmittel und Emulgatoren in Konzentrationen von 0,5 bis 30 Gewichtsprozent, die Verdicker in einer Menge von 0,1 bis 25 Gewichtsprozent und die Pflegestoffe in einer Konzentration von 0,1 bis 5,0 Gewichtsprozent.

Die in den hier beschriebenen Haarfärbemitteln enthaltenen Verbindungen der Formel (I) sind toxikologisch und dermatologisch unbedenklich und ermöglichen Haarfärbungen mit einer hervorragenden Stabilität gegenüber Säuren und sauren Zubereitungen.

Hinsichtlich der färberischen Möglichkeiten bieten diese Haarfärbemittel je nach Art und Zusammensetzung der Farbkomponenten eine breite Palette verschiedener Farbnuancen, die sich von natürlichen Farbtönen bis hin zu hochmodischen, leuchtenden Nuancen erstreckt.

Gegenstand dieser Anmeldung sind weiterhin die neuen 4-[4'-[Bis-(β-hydroxy-ethyl)-amino]-phenylazo]-benzolsulfonsäureamide der Formel (II)

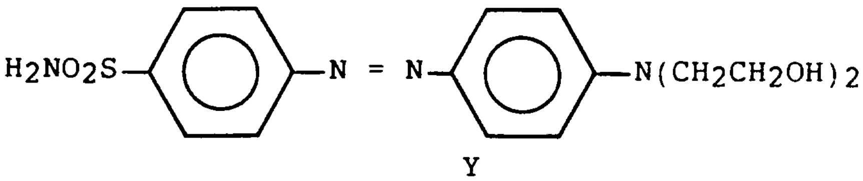

(II),

wobei Y Fluor, Chlor, Brom, einen $C_2$- bis $C_3$-Alkylrest oder einen $C_1$- bis $C_3$-Alkoxyrest darstellt.

Unter den Verbindungen der Formel (II) sind insbesondere zu nennen:
4-[4'-[Bis-(β-hydroxyethyl)amino]-2'-methoxy-phenylazo]-benzolsulfonsäureamid,
4-[4'-[Bis-(β-hydroxyethyl)amino]-2'-fluor-phenylazo]-benzolsulfonsäureamid,
4-[4'-[Bis-(β-hydroxyethyl)amino]-2'-chlor-phenylazo]-benzolsulfonsäureamid,
4-[4'-[Bis-(β-hydroxyethyl)amino]-2'-ethyl-phenylazo]-benzolsulfonsäureamid.

Diese neuen Verbindungen der Formel (II) lassen sich ebenso wie die Verbindungen der Formel (I) durch ein einstufiges Verfahren gemäß der folgenden Reaktionsgleichung herstellen (Y = F, Cl, Br, $C_2$- bis $C_3$-Alkyl oder $C_1$- bis $C_3$-Alkoxy):

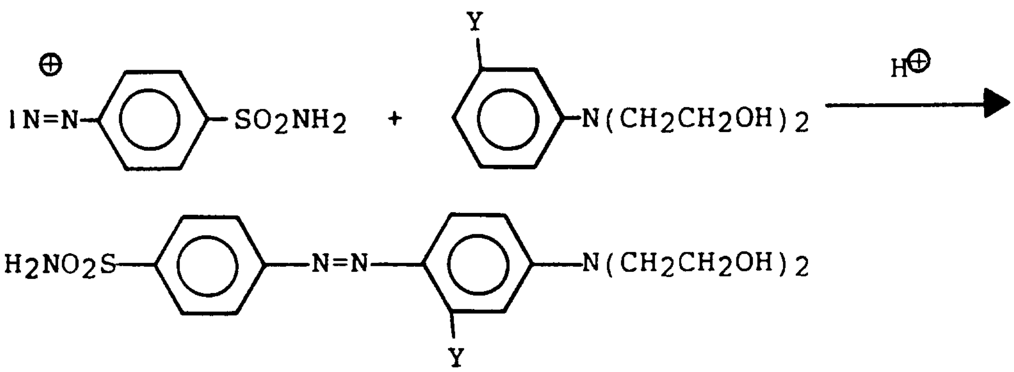


Das Herstellungsverfahren wird im einzelnen wie folgt durchgeführt:
Eine wäßrige Lösung des Diazoniumsalzes des 4-Aminobenzolsulfonsäureamides wird unter Kühlung zu einer äquimolaren Menge des N,N-(β-Hydroxyethyl)-anilins getropft. Sodann wird die Reaktionsmischung 24 Stunden lang bei Raumtemperatur gerührt und anschließend mit Ammoniak neutralisiert.

Der erhaltene Niederschlag wird abfiltriert, mit Wasser gewaschen und über Calciumchlorid getrocknet.

Zur Herstellung der Hydrochloride werden die Verbindungen der Formel (II) in Ethanol gelöst und mit einer 3-molaren Salzsäure versetzt. Anschließend werden die Hydrochloride unter Verwendung von Diethylether aus der Reaktionsmischung ausgefällt.

Die nachfolgenden Beispiele sollen der Gegenstand der vorliegenden Erfindung näher erläutern, ohne ihn auf diese Beispiele zu beschränken.

**Herstellungsbeispiele**

**Beispiel 1 bis 4:** Herstellung von 4-[4'-[Bis-(β-hydroxyethyl)-amino]-phenylazo]-benzolsulfonsäureamiden der Formel (I)

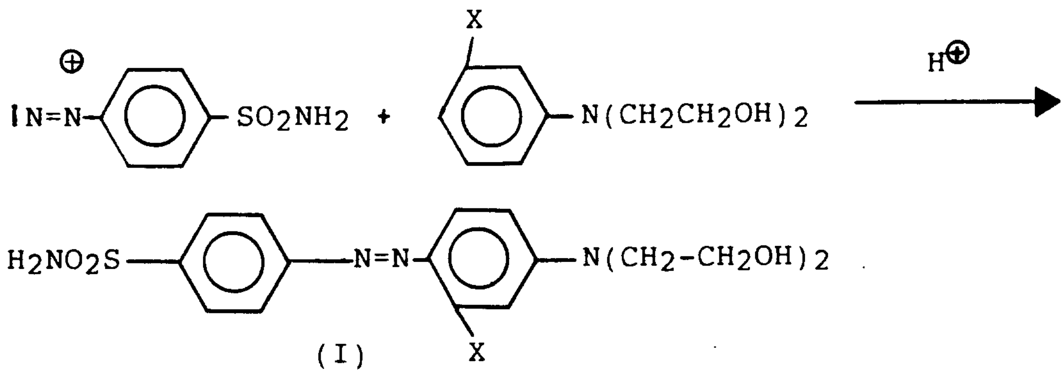

(I)

6,9 g (0,04 mol) 4-Amino-benzolsulfonsäureamid werden in einer Mischung aus 20 g Wasser, 60 g zerstoßenem Eis und 6,8 ml konzentrierter Salzsäure mit 3,0 g (0,044 mol) Natriumnitrit bei O Grad Celsius diazotiert. Die erhaltene Lösung des Diazoniumsalzes wird filtriert und das Filtrat wird innerhalb von einer Stunde unter Rühren tropfenweise zu einer gekühlten Lösung von 0,04 mol des entsprechenden in 3-Stellung substituierten N,N-Bis-(β-hydroxyethyl)-anilins in 32 ml Wasser und 6,8 ml konzentrierter Salzsäure gegeben.

Anschließend wird die Reaktionsmischung 24 Stunden lang bei Raumtemperatur gerührt und sodann mit Ammoniak neutralisiert (pH = 7,5). Die ausgefallene Azoverbindung wird abfiltriert, mit Wasser gewaschen und über Calciumchlorid getrocknet.

Zur Herstellung der Hydrochloride werden die Benzolsulfonsäureamide der Formel (I) in Ethanol gelöst und mit 3-molarer HCl versetzt. Die Hydrochloride werden sodann mit Diethylether ausgefällt.

Es werden die folgenden Reaktionsprodukte erhalten:

**Beispiel 1:** 4-[4'-[Bis-(β-hydroxyethyl)-amino]-2'-ethyl-phenylazo]-benzolsulfonsäureamid

Ausbeute: 13,6 g ( 87 % der Theorie)
Schmelzpunkt: 196 bis 197 °C (rotes, kristallines Pulver)

| CHN-Analyse: | ($C_{18}H_{24}N_4O_4S$) | | |
|---|---|---|---|
| (MG = 392,48) | % C | % H | % N |
| berechnet: | 55,09 | 6,16 | 14,28 |
| gefunden: | 54,86 | 6,04 | 14,03 |

**Beispiel 2:** 4-[4'-[Bis-(β-hydroxyethyl)-amino]-2'-methoxy-phenylazo]-benzolsulfonsäureamid-Hydrochlorid

Ausbeute: 6,0 g (35 % der Theorie)
Schmelzpunkt: 86 bis 89 °C unter Zersetzung (dunkelbraunes Pulver)

| CHNCl-Analyse: | ($C_{17}H_{22}N_4O_5S$ x HCl) | | | |
|---|---|---|---|---|
| (MG = 430,91) | % C | % H | % N | % Cl |
| berechnet: | 47,38 | 5,38 | 13,00 | 8,23 |
| gefunden: | 46,58 | 5,45 | 11,85 | 6,72 |

**Beispiel 3:** 4-[4'-[Bis-(β-hydroxyethyl)-amino]-2'-fluor-phenylazo]-benzolsulfonsäureamid

Ausbeute: 8,8 g (53 % der Theorie)
Schmelzpunkt: 173 bis 175 °C (oranges Pulver)

| CHNS-Analyse: | ($C_{16}H_{19}FN_4O_4S$) | | | |
|---|---|---|---|---|
| (MG = 418,87) | % C | % H | % N | % S |
| berechnet: | 50,25 | 5,01 | 14,65 | 8,38 |
| gefunden: | 49,27 | 5,13 | 13,81 | 8,10 |

**Beispiel 4**: 4-[4'-[Bis-(β-hydroxyethyl)-amino]-2'-chlor-phenylazo]-benzolsulfonsäureamid-Hydrochlorid

Ausbeute: 10,8 g (68 % der Theorie)
Schmelzpunkt: 181 bis 183 °C unter Zersetzung (dunkelrotes Pulver)

| CHNCl-Analyse: | ($C_{16}H_{19}ClN_4O_4S$ x HCl) | | | |
|---|---|---|---|---|
| (MG = 398,86) | % C | % H | % N | % Cl |
| berechnet: | 44,15 | 4,63 | 12,87 | 16,29 |
| gefunden: | 44,25 | 4,40 | 12,94 | 13,38 |

**Beispiel 5:** 4-[4'-[Bis-(β-hydroxyethyl)-amino]-phenylazo]-benzolsulfonsäureamid-Hemihydrat

Ausbeute: 9,7 g (65 % der Theorie)
Schmelzpunkt: 181 bis 183 °C (orange Blättchen)

| CHNS-Analyse: | ($C_{16}H_{20}N_4O_4S$ x $1/2H_2O$) | | | |
|---|---|---|---|---|
| (MG = 373,43) | % C | % H | % N | % S |
| berechnet: | 51,46 | 5,67 | 15,00 | 8,59 |
| gefunden: | 51,53 | 5,56 | 14,71 | 8,59 |

**Beispiel 6:** 4-[4'-[Bis-(β-hydroxyethyl)-amino]-phenylazo]-benzolsulfonsäureamid-Hydrochlorid

Schmelzpunkt: 185 bis 186 °C unter Zersetzung (rotes Pulver)

| CHNCl-Analyse: | ($C_{16}H_{20}N_4O_4S$ x HCl) | | | |
|---|---|---|---|---|
| (MG = 400,88) | % C | % H | % N | % Cl |
| berechnet: | 47,94 | 5,28 | 13,98 | 8,84 |
| gefunden: | 47,21 | 5,17 | 13,39 | 9,06 |

**Beispiel 7:** 4-[4'-[Bis-(β-hydroxyethyl)-amino]-2'-methyl-phenylazo]-benzolsulfonsäureamid-Hydrochlorid

Ausbeute: 12,4 g (75 % der Theorie)
Schmelzpunkt: 95 bis 98 °C unter Zersetzung (braunes Pulver)

| CHNCl-Analyse: | ($C_{17}H_{22}N_4O_4S$ x HCl) | | | |
|---|---|---|---|---|
| (MG = 414,91) | % C | % H | % N | % Cl |
| berechnet: | 49,21 | 5,59 | 13,50 | 8,54 |
| gefunden: | 48,30 | 5,65 | 12,75 | 7,84 |

**Beispiele für Haarfärbemittel:**

**Beispiel 8**: Haarfärbelösung

| | |
|---|---|
| 0,3 g | 4-[4'-[Bis-(β-hydroxyethyl)-amino]-phenylazo]-benzolsulfonsäureamid der allgemeinen Formel (I) |
| 2,0 g | Laurylalkoholdiglykolethersulfat-Natriumsalz (28-prozentige wäßrige Lösung) |
| 2,0 g | Ammoniak (25-prozentige wäßrige Lösung) |
| 10,0 g | Isopropanol |
| 85,7 g | Wasser |
| 100,0 g | |

Gebleichtes Humanhaar wird 20 Minuten lang bei Raumtemperatur mit der vorstehenden Haarfärbelösung behandelt. Danach wird das Haar mit Wasser gespült und getrocknet. Das Haar ist wie in nachfolgender Tabelle 1 angegeben gefärbt.

Tabelle 1

| Haarfärbungen | |
|---|---|
| Verwendetes 4-[4'-[Bis-(β-hydroxyethyl)-amino]-phenylazo]-benzolsulfonsäureamid der Formel (I) | Haarfarbe |
| 4-[4'-[Bis-(β-hydroxyethyl)-amino]-2'-fluor-phenylazo]-benzolsulfonsäureamid | orange |
| 4-[4'-[Bis-(β-hydroxyethyl)-amino]-2'-chlor-phenylazo]-benzolsulfonsäureamid-Hydrochlorid | orange |
| 4-[4'-[Bis-(β-hydroxyethyl)-amino]-2'-ethyl-phenylazo]-benzolsulfonsäureamid | orange |
| 4-[4'-[Bis-(β-hydroxyethyl)-amino]-2'-methoxy-phenylazo] -benzolsulfonsäureamid | rotorange |
| 4-[4'-[Bis-(β-hydroxyethyl)-amino]-2'-methyl-phenylazo]-benzolsulfonsäureamid | hellrot |

**Beispiel 9:** Haarfärbecreme

| | |
|---|---|
| 0,04 g | 4-[4'-[Bis-(β-hydroxyethyl)-amino]phenylazo]-benzolsulfonsäureamid-Hemihydrat |
| 0,30 g | $N^1$,$N^4$,$N^4$-Tris-(β-hydroxyethyl)-2-nitro-p-phenylendiamin |
| 0,03 g | 1-Nitro-4-[(2-ureidoethyl)amino]-benzol |
| 0,02 g | Disperse Blue 3 (C.I. 61 505) |
| 0,02 g | Disperse Violet 1 (C.I. 61 100) |
| 0,02 g | 4-[(2-Hydroxyethyl)amino]-2-nitroanilin |
| 7,00 g | Cetylalkohol |
| 2,00 g | Laurylalkohol-diglykolethersulfat-Natriumsalz (28-prozentige wäßrige Lösung) |
| 0,20 g | Ammoniak (25-prozentige wäßrige Lösung) |
| 0,10 g | p-Hydroxybenzoesäuremethylester |
| 90,27 g | Wasser |
| 100,00 g | |

50 g des vorstehenden Haarfärbemittels werden auf weiße menschliche Haare aufgetragen und nach einer Einwirkungszeit von 20 Minuten mit Wasser ausgespült.

Anschließend wird das Haar getrocknet. Das so behandelte Haar besitzt einen natürlichen Braunton.

**Beispiel 10:** Haarfärbelösung

| | |
|---|---|
| 0,05 g | 4-[4'-[Bis-(β-hydroxyethyl)-amino]-2'-chlor-phenylazo]-benzolsulfonsäureamid-Hydrochlorid |
| 0,05 g | 1,4-Diaminoanthrachinon |
| 0,10 g | $N^1$,$N^4$,$N^4$-Tris-(β-hydroxyethyl)-2-nitro-p-phenylendiamin |
| 0,50 g | Hydroxyethylcellulose |
| 5,00 g | Laurylalkohol-diglykolethersulfat-Natriumsalz (28-prozentige wäßrige Lösung) |
| 10,00 g | Ammoniak (25-prozentige wäßrige Lösung) |
| 10,00 g | Isopropanol |
| 74,30 g | Wasser |
| 100,00 g | |

Gebleichtes menschliches Naturhaar wird 20 Minuten lang bei Raumtemperatur mit der vorstehenden Haarfärbelösung behandelt. Sodann werden die Haare mit Wasser gespült und mit einem Haarshampoo gewaschen. Nach dem Trocknen besitzen die Haare eine blond-aschfarbene Färbung.

**Beispiel 11**: Vergleichsversuche zur Säurebeständigkeit

Zum Vergleich der Säurestabilität der erfindungsgemäßen 4-[4'-[Bis-(β-hydroxyethyl)-amino]-phenylazo]-benzolsulfonsäureamide mit aus dem Stand der Technik bekannten 4'-Amino-4-[bis-(β-hydroxyethyl)amino]-azobenzolen wurden Naturhaarsträhnen in der in Beispiel 8 beschriebenen Weise gefärbt, wobei folgende Haarfärbemittel verwendet wurden:

(i) ein Mittel gemäß Beispiel 8, in dem die erfindungsgemäßen 4-[4'-[Bis-(β-hydroxyethyl)amino]-phenylazo]-benzolsulfonsäureamide durch die gleiche Menge an 4'-Amino-4-[bis-(β-hydroxyethyl)amino]-azobenzol ersetzt wurden,
(ii) ein Mittel gemäß Beispiel 8 (Verbindung gemäß Tabelle 2 mit X=H; $R=SO_2NH_2$),
(iii) ein Mittel gemäß Beispiel 8 (Verbindung gemäß Tabelle 2 mit $X=CH_3$; $R=SO_2NH_2$),
(iv) ein Mittel gemäß Beispiel 8 (Verbindung gemäß Tabelle 2 mit $X=OCH_3$; $R=SO_2NH_2$),
(v) ein Mittel gemäß Beispiel 8 (Verbindung gemäß Tabelle 2 mit X=F; $R=SO_2NH_2$),
(vi) ein Mittel gemäß Beispiel 8 (Verbindung gemaß Tabelle 2 mit X=Cl; $R=SO_2NH_2$),
(vii) ein Mittel gemäß Beispiel 8 (Verbindung gemaß Tabelle 2 mit $X=C_2H_5$; $R=SO_2NH_2$)

Anschließend wurden die so gefärbten Haarsträhnen mit 1n-Salzsäure eine Minute lang behandelt, sodann mit einem sauer eingestellten (pH=5,5) Shampoo gewaschen und getrocknet.

Die Lab-Farbwerte der erhaltenen gefärbten Haarsträhnen wurden mit einem Minolta-Farbmeßgerät, Typ CR-200 bestimmt. Die ermittelten Lab-Farbwerte sind in der nachfolgenden Tabelle 2 zusammengefaßt:

**Tabelle 2:** Farbmeßwerte der Waschversuche mit 1n-Salzsäure

| 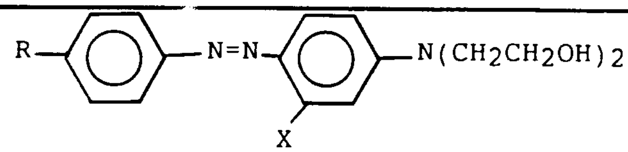 | | | |
|---|---|---|---|
| R | X | unbehandelt | 1n-Salzsäure |
| (i) $NH_2$ | H | L 58,8<br>a 41,7<br>b 77,3 | 37,5<br>6,0<br>38,1 |
| (ii) $SO_2NH_2$ | H | L 58,1<br>a 38,1<br>b 67,1 | 63,4<br>38,4<br>74,0 |
| (iii) $SO_2NH_2$ | $CH_3$ | L 53,2<br>a 54,0<br>b 61,6 | 56,4<br>54,8<br>64,4 |
| (iv) $SO_2NH_2$ | $OCH_3$ | L 54,5<br>a 52,3<br>b 61,2 | 58,4<br>51,3<br>65,4 |
| (v) $SO_2NH_2$ | F | L 64,6<br>a 37,0<br>b 78,6 | 66,0<br>34,9<br>80,8 |
| (vi) $SO_2NH_2$ | Cl | L 53,2<br>a 49,6<br>b 62,1 | 58,0<br>48,4<br>69,4 |
| (vii) $SO_2NH_2$ | $C_2H_5$ | L 63,5<br>a 30,8<br>b 56,2 | 66,1<br>36,8<br>51,7 |

Aus Tabelle 2 ist ersichtlich, daß die Farbmeßwerte der mit dem erfindungsgemäßen Mittel (ii-vii) behandelten Haarsträhnen durch Behandlung mit Säuren in wesentlich geringerem Maße herabgesetzt werden als dies bei mit der bekannten Verbindung (i) behandelten Haarsträhnen der Fall ist.

Die im Vergleich zum Stand der Technik wesentlich verbesserte Säurestabilität des erfindungsgemäßen Mittels wird noch deutlicher, wenn die Gesamtfarbänderung ΔY nach Anderson (vgl. US-PS 5 000 755) gemäß der folgenden Formel berechnet wird:

$$\Delta Y = \sqrt{(\Delta L)^2 + (\Delta a)^2 + (\Delta b)^2}$$

Die ermittelten Werte für die Gesamtfarbänderung ΔY sind in Tabelle 3 zusammengefaßt:

**Tabelle 3:** Gesamtfarbänderung ΔY

| 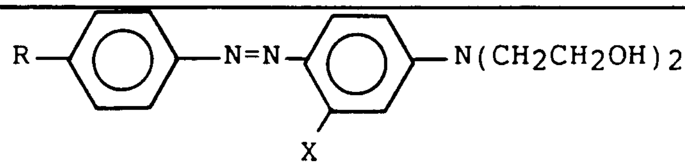 | | |
|---|---|---|
| R | X | 1n-Salzsäure |
| (i) $NH_2$ | H | 57,1 |
| (ii) $SO_2NH_2$ | H | 8,7 |
| (iii) $SO_2NH_2$ | $CH_3$ | 4,3 |
| (iv) $SO_2NH_2$ | $OCH_3$ | 5,8 |
| (v) $SO_2NH_2$ | F | 3,3 |
| (vi) $SO_2NH_2$ | Cl | 8,8 |
| (vii) $SO_2NH_2$ | $C_2H_5$ | 7,9 |

Die mit dem erfindungsgemäßen Mittel (ii-vii) behandelten Haarsträhnen zeigen bei der Einwirkung von 1n-Salzsäure eine wesentliche geringere Gesamtfarbänderung (ΔY) als die mit dem bekannten Mittel (i) behandelten Haarsträhnen.

Alle Prozentangaben stellen, soweit nicht anders vermerkt, Gewichtsprozente dar.

**Patentansprüche**

1. Mittel zum Färben von Haaren mit einem Gehalt an für Haarfärbemittel üblichen Zusätzen, dadurch gekennzeichnet, daß es mindestens ein 4-[4'-[Bis-(β-hydroxyethyl)-amino]-phenylazo]-benzolsulfonsäuramid der allgemeinen For-

mel (I)

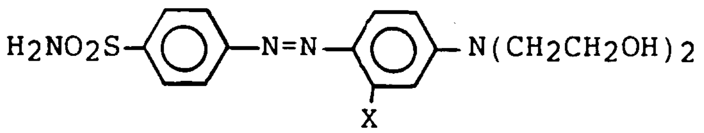

(I),

wobei X Wasserstoff, Fluor, Chlor, Brom, einen $C_1$– bis $C_3$-Alkylrest oder einen $C_1$– bis $C_3$–Alkoxyrest bedeutet, enthält.

2. Mittel nach Anspruch 1, dadurch gekennzeichnet, daß das 4-[4'-[Bis-(β-hydroxyethyl)-amino]-phenylazo]-benzolsulfonsäureamid ausgewählt ist aus Verbindungen der Formel (I), wobei X Wasserstoff, Fluor, Chlor, Methyl oder Methoxy bedeutet.

3. Mittel nach Anspruch 1 oder 2, dadurch gekennzeichnet, daß das 4-[4'-[Bis-(β-hydroxyethyl)-amino]-phenylazo]-benzolsulfonsäuramid der Formel (I) in einer Menge von 0,01 bis 5 Gewichtsprozent enthalten ist.

4. Mittel nach einem der Ansprüche 1 bis 3, dadurch gekennzeichnet, daß es einen pH-Wert von 3 bis 12 aufweist.

5. Mittel nach einem der Ansprüche 1 bis 4, dadurch gekennzeichnt, daß es in Form einer wäßrigen oder wäßrig-alkoholischen Lösung, einer Creme, eines Geles, einer Emulsion oder eines Schaumes vorliegt.

6. Mittel nach einem der Ansprüche 1 bis 5, dadurch gekennzeichnet, daß es zusätzlich einen oder mehrere direkt auf das Haar aufziehende Farbstoffe enthält.

7. Mittel nach Anspruch 6, dadurch gekennzeichnet, daß der zusätzlich enthaltene direkt auf das Haar aufziehende Farbstoff ausgewählt ist aus 2-Amino-6-chlor-4-nitrophenol, 2-Amino-4,6-dinitrophenol, 4-[(β-Hydroxyethyl)amino]-2-nitroanilin, 2-Chlor-6-ethylamino-4-nitro-benzol, $N^1$,$N^4$,$N^4$-Tris(β-hydroxyethyl)-p-phenylendiamin, 4-[Bis-(β-hydroxyethyl)amino]-1-methylamino-2-nitro-benzol, 1-[(2,3-Dihydroxypropyl)amino]-4-ethyl-[(2-hydroxyethyl)amino]-2-nitrobenzol, 1-[(2,3-Dihydroxypropyl)amino]-4-dimethylamino-2-nitrobenzol, 1-[(2,3-Dihydroxypropyl)amino]-2-nitro-4-pyrrolidinobenzol, 4-[Bis-(β-hydroxyethyl)amino]-1-[(3-hydroxypropyl)amino]-2-nitrobenzol, 2-Amino-4-nitrophenol, 2-Nitro-1,4-diaminobenzol, 2-Amino-5-nitrophenol, 4-(β-Hydroxyethyl)amino-3-nitrophenol, 1-(β-Hydroxyethyl)amino-2-amino-4-nitrobenzol, 4-(β-Ureidoethyl)-amino-nitrobenzol, 4-(2',3'-Dihydroxypropyl)amino-3-nitrotrifluormethylbenzol, 1,4-Bis-[(β-hydroxyethyl)-amino]-4-N-ethyl-2-nitrobenzol, 4-(β-Hydroxyethyl)-amino-3-nitrotoluol, 2,5-Bis-[(β-hydroxyethyl)amino]-nitrobenzol, 2-(β-Hydroxyethyl)-amino-4,6-dinitrophenol, 1-Amino-4-(2',3'-dihydroxypropyl)amino-2-nitro-5-chlorbenzol, 1-Amino-2-nitro-4-[bis-(β-hydroxyethyl)amino]-benzol, Basic Violet 1 (C.I. 42 535), Basic Violet 14 (C.I. 42 510), Basic Violet 2 (C.I. 42 520), Acid Brown 4 (C.I. 14 805), 1-[(β-Hydroxyethyl)amino]-4-methylamino-anthrachinon, Disperse Blue 23 (C.I. 61 545), 1,4-Diamino-anthrachinon, 1,4,5,8-Tetraaminoanthrachinon.

8. Mittel nach einem der Ansprüche 1 bis 7, dadurch gekennzeichnet, daß es in Form eines Haarfärbemittels mit zusätzlicher Haarfestigung vorliegt und mindestens ein in der Kosmetik überlicherweise verwendetes Polymerisat oder natürliches Polymer enthält.

9. Mittel nach Anspruch 8, dadurch gekennzeichnet, daß das Polymerisat oder das natürliche Polymer ausgewählt ist aus Polyvinylpyrrolidon, Polyvinylacetat, Polyvinylalkohol, Polyacrylsäure, Polymethacrylsäure sowie basischen Polymerisaten der Ester der Polyacrylsäure oder Polymethacrylsäure mit Aminoalkoholen, deren Salzen oder Quaternisierungsprodukten, Polyarcylnitril, Polyvinyllactamen oder Copolymerisaten aus diesen Verbindungen, Chitosan und Chitosanderivaten.

**10.** 4-[4'-[Bis-(β-hydroxyethyl)-amino]-phenylazo]-benzolsulfonsäureamide der Formel (II)

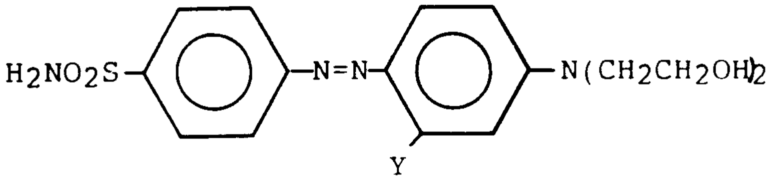

(II),

wobei Y Fluor, Chlor, Brom, einen $C_2$_ bis $C_3$_ Alkylrest oder einen $C_1$_ bis $C_3$_Alkoxyrest darstellt.

**11.** 4-[4'-[Bis-(β-hydroxyethyl)amino]-2'-methoxyphenylazo]-benzolsulfonsäureamid.

**12.** 4-[4'-[Bis-(β-hydroxyethyl)amino]-2'-fluor-phenylazo]-benzolsulfonsäureamid.

**13.** 4-[4'-[Bis-(β-hydroxyethyl)amino]-2'-chlor-phenylazo]-benzolsulfonsäureamid.

**14.** 4-[4'[Bis-(β-hydroxyethyl)amino]-2'-ethyl-phenylazo]-benzolsulfonsäureamid.

## Claims

1. Agent for the colouring of hair with additives that are conventional for hair colouring agents, characterised in that it contains at least one 4-[4'-[bis-(β-hydroxyethyl)-amino]-phenylazo]-benzene sulphonic acid amide of the general formula (I)

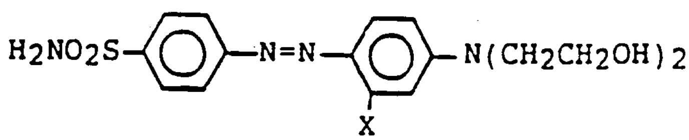

(I),

in which X represents hydrogen, fluorine, chlorine, bromine, a $C_1$ to $C_3$ alkyl residue or a $C_1$ to $C_3$ alkoxy residue.

2. Agent according to Claim 1, characterised in that the 4-[4'-[bis-(β-hydroxyethyl)-amino]-phenylazo]-benzene sulphonic acid amide is selected from compounds of formula (I), wherein X represents hydrogen, fluorine, chlorine, methyl or methoxy.

3. Agent according to Claim 1 or Claim 2, characterised in that the 4-[4'-[bis-(β-hydroxyethyl)-amino]-phenylazo]-benzene sulphonic acid amide of the formula (I) is contained in an amount of from 0.01 to 5 weight %.

4. Agent according to any one of Claims 1 to 3, characterised in that it has a pH value of from 3 to 12.

5. Agent according to any one of Claims 1 to 4, characterised in that it is in the form of an aqueous or aqueous alcoholic solution, a cream, a gel, an emulsion or a foam.

6. Agent according to any one of Claims 1 to 5, characterised in that it further contains one or more dyes acting directly on the hair.

7. Agent according to Claim 6, characterised in that the dye which is additionally contained and which acts directly on the hair is selected from among 2-amino-6-chloro-4-nitrophenol, 2-amino-4,6-dinitrophenol, 4-[(β-hydroxyethyl)amino]-2-nitroaniline, 2-chloro-6-ethylamino-4-nitro-benzene, $N^1,N^4,N^4$-tris(β-hydroxyethyl)-p-phenylenediamine, 4-[bis-(β-hydroxyethyl)amino]-1-methylamino-2-nitrobenzene, 1-[(2,3-dihydroxypropyl)amino]-4-ethyl-[(2-hydroxyethyl)amino]-2-nitrobenzene, 1-[(2,3-dihydroxypropyl)amino]-4-dimethylamino-2-nitrobenzene, 1-[(2,3-dihydroxypropyl)amino]-2-nitro-4-pyrrolidinobenzene, 4-[bis-(β-hydroxyethyl)amino]-1-[(3-hydroxypropyl)amino]-2-nitrobenzene, 2-amino-4-nitrophenol, 2-nitro-1,4-diaminobenzene, 2-amino-5-nitrophenol, 4-(β-hyddroxyethyl)amino-3-nitrophenol, 1-(β-hydroxyethyl)amino-2-amino-4-nitrobenzene, 4-(β-ureidoethyl)-amino-nitro-benzene, 4-(2',3'-dihydroxypropyl)amino-3-nitrotrifluoromethylbenzene, 1,4-bis-[(β-hydroxyethyl)-amino]-4-N-ethyl-2-nitrobenzene, 4-(β-hydroxyethyl)-amino-3-nitrotoluene, 2,5-bis-[(β-hydroxyethyl)amino]-nitrobenzene, 2-(β-hydroxyethyl)-amino-4,6-dinitrophenol, 1-amino-4-(2',3'-dihydroxypropyl)amino-2-nitro-5-chlorobenzene, 1-amino-2-nitro-4-[bis-(β-hydroxyethyl)amino]-benzene, Basic Violet 1 (C.I. 42 535), Basic Violet 14 (C.I. 42 510), Basic Violet 2 (C.I. 42 520), Acid Brown 4 (C.I. 14 805), 1-[(β-hydroxyethyl)amino]-4-methylaminoanthraquinone, Disperse Blue 23 (C.I. 61 545), 1,4-diamino-anthraquinone, 1,4,5,8-tetraaminoanthraquinone.

8. Agent according to any one of Claims 1 to 7, characterised in that it is in the form of a hair colouring agent with additional hair fixing, and contains at least one polymerisate or natural polymer which is conventionally used in the cosmetics industry.

9. Agent according to Claim 8, characterised in that the polymerisate or the natural polymer is selected from among polyvinylpyrrolidone, polyvinylacetate, polyvinyl alcohol, polyacrylic acid, polymethacrylic acid and basic polymerisates of the esters of polyacrylic acid or polymethacrylic acid with amino alcohols, the salts or quaternary products thereof, polyacrylnitrile, polyvinyllactams or copolymers of these compounds, chitosan and chitosan derivatives.

10. 4-[4'-[bis-(β-hydroxyethyl)-amino]-phenylazo]-benzene sulphonic acid amide of the formula (II)

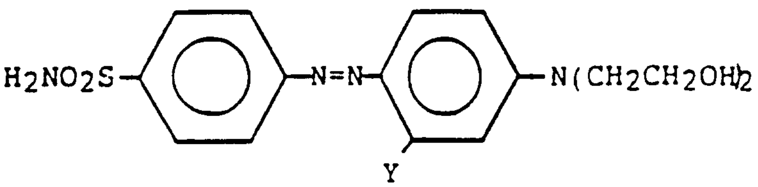

(II),

wherein Y represents fluorine, chlorine, bromine, a $C_2$ to $C_3$ alkyl residue or a $C_1$ to $C_3$ alkoxy residue.

11. 4-[4'-[bis-(β-hydroxyethyl)amino]-2'-methoxy-phenylazo]-benzene sulphonic acid amide.

12. 4-[4'-[bis-(β-hydroxyethyl)amino]-2'-fluorine-phenylazo]-benzene sulphonic acid amide.

13. 4-[4'-[bis-(β-hydroxyethyl)amino]-2'-chloro-phenylazo]-benzene sulphonic acid amide.

14. 4-[4'-[bis-(β-hydroxyethyl)amino]-2'-ethyl-phenylazo]-benzene sulphonic acid amide.

**Revendications**

1. Produit de teinture des cheveux comportant une teneur en colorants pour cheveux avec les additifs usuels, caractérisé en ce qu'il contient au moins un ami de de l'acide 4-[4'-[bis-(β-hydroxyéthyl)-amino]-phénylazo] benzolsulfonique de formule générale (I)

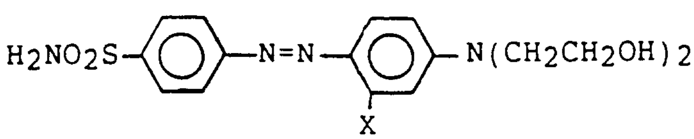

(I),

dans laquelle X est l'hydrogène, le fluor, le chlore, le brome, un résidu alkyl en $C_1$ à $C_3$ ou un résidu alcoxy en $C_1$ à $C_3$.

2. Produit selon la revendication 1, caractérisé en ce que l'amide de l'acide 4-[4'-[bis-(β-hydroxyéthyl)-amino]-phénylazol benzolsulfonique est choisi parmi les combinaisons de formule (I) dans laquelle X est l'hydrogène, le fluor, le chlore, le radical méthyl ou méthoxy.

3. Produit selon la revendication 1 ou 2, caractérisé en ce que l'amide de l'acide 4-[4'-[bis-(β-hydroxyéthyl)-amino]-phénylazo] benzolsulfonique de formule (I) est contenu en une quantité allant de 0,01 à 5 pourcent en poids.

4. Produit selon l'une des revendications 1 à 3, caractérisé en ce qu'il a un pH compris entre 3 et 12.

5. Produit selon l'une des revendications 1 à 4, caractérisé en ce qu'il se pésente sous forme d'une solution aqueuse ou alcoolo-aqueuse, d'une crème d'un gel d'une émulsion ou d'une mousse.

6. Produit selon l'une des revendications 1 à 5, caractérisé en ce qu'il contient en plus un ou plusieurs colorants agissant directement sur les cheveux.

7. Produit selon la revendication 6, caractérisé en ce que le colorant contenu en plus et agissant directement sur les cheveux est choisi parmi :
2-amino-6-chloro-4-nitrophénol,
2-amino-4,6-dinitrophénol,
4-[β(-hydroxyéthyl)amino]-2-nitroaniline,
2-chloro-6-éthylamino-4-nitrobenzène,
$N^1$,$N^4$,$N^4$-tris(β-hydroxyéthyl)-p-phénylènediamine,
4-[bis-(β-hydroxyéthyl)amino]-1-méthylamino-2-nitrobenzène,
1-[(2, 3-dihydroxypropyl)amino]-4-éthyl-[(2-hydroxyéthyl)amino]-2-nitrobenzène
1-[(2, 3-dihydroxypropyl)amino]-4-diméthylamino-2-nitrobenzène,
1-[(2,3-dihydroxypropyl)amino]-2-nitro-4-pyrrolidinobenzène,
4-[bis-(β-hydroxyéthyl)amino]-1-[(3-hydroxypropyl)amino]-2-nitrobenzène,
2-amino-4-nitrophénol,
2-nitro-1,4-diaminobenzène,
2-amino-5-nitrophénol,
4-(β-hydroxyéthyl)amino-3-nitrophénol,
1-(β-hydroxyéthyl)amino-2-amino-4-nitrobenzène,
4-(β-uréidoéthyl)amino-nitrobenzène
4-(2', 3'-dihydorxypropyl)amino-3-nitrotrifluométhylbenzène,
1,4-bis-[(β-hydroxyéthyl)amino]-4-N-éthyl-2-nitrobenzène,
4-(β-hydroxyéthyl)amino-3-nitrotoluène,
2,5-bis[(β-hydroxyéthyl)amino]-nitrobenzène
2-(β-hydroxyéthyl)amino]-4,6-dinitrophénol,
1-amino-4-(2', 3'-dihydroxyproyl)amino-2-nitro-5-chlorobenzène,
1-amino-2-nitro-4-[bis(β-hydroxyéthyl)amino]-benzène, Basic Violet 1 (C.I. 42 535) Basic Violet 14 (C.I. 42 510) Basic Violet 2 (C.I. 42 520) Acid Brown 4 (C.I. 14 805) 1-[(β-hydroxyéthyl)amino]-4-méthylaminoanthrachinone, Disperse Blue 23 (C.I. 61 545) 1,4-diamino-antrachinone, 1,4,5,8-tétraaminoantrachinone.

8. Produit selon l'une des revendications 1 à 7, caractérisé en ce qu'il se présente sous la forme d'un colorant pour cheveux avec un effet supplémentaire fixateur et contient au moins un polymérisat ou un polymère naturel usuellement utilisé en cosmétique.

9. Produit selon la revendication 8, caractérisé en ce que le polymérisat ou le polymère naturel est sélectionné parmi la polyvinyl pyrrolidone, l'acétate de polyvinyle, l'alcool polyvinylique, l'acide polyacrylique, l'acide polyméthacrylique, ainsi que les polymérisats basiques des esters de l'acide polyacrylique ou de l'acide polyméthacrylique, avec des aminoalcools leurs sels ou produits de quaternisation, le polyarcylnitrile, le polyvinyllactamene ou les copolymérisats issus de ces combinaisons, le chitosane et les dérivés du chitosane.

10. Amide de l'acide 4-[4'-[bis-(β-hydroxyéthyl)-amino]-phénylazo] benzolsulfonique de formule (II)

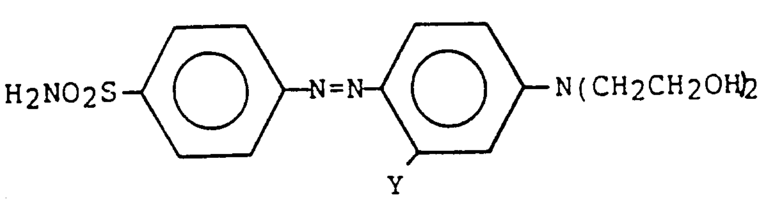

(II),

dans laquelle Y est le fluor, le chlore, le brome, un résidu alkyl en $C_2$ à $C_3$ ou un résidu alkoxy en $C_1$ à $C_3$.

11. Amide de l'acide 4-[4'-[bis-(β-hydroxyéthyl)-amino]-2'-méthoxy-phénylazo] benzolsulfonique.

12. Amide de l'acide 4-[4'-[bis-(β-hydroxyéthyl)-amino]-2'-fluoro-phénylazo] benzolsulfonique.

**13.** Ami de de l'acide 4-[4'-[bis-(β-hydroxyéthyl)-amino]-2'-chloro-phénylazo] benzolsulfonique.

**14.** Ami de de l'acide 4-[4'-[bis-(β-hydroxyéthyl)-aminol-2'-éthyl-phénylazo] benzolsulfonique.